# EUROPEAN PATENT SPECIFICATION

(11) **EP 1 469 301 B1**
(45) Date of publication and mention of the grant of the patent: **24.05.2006**
(21) Application number: 03425249.4
(22) Date of filing: 18.04.2003
(51) Int. Cl.: G01N 21/95, G01N 21/89, B07C 5/342

(54) **An apparatus for optically checking the exterior quality of bulbous fruits and vegetable products of various shapes and sizes**
Gerät zur optischen Überprüfung der Qualität der Aussenoberfläche von gewölbt geformtem Obst und Gemüse verschiedenster Form und Grösse
Dispositif de contrôle optique de la qualité extérieure des fruits et légumes bulbeux de forme et taille diverses

(43) Date of publication of application: 20.10.2004
(73) Proprietor: S.A.M.M.O. S.p.A., I-47023 Cesena (FC) (IT)
(72) Inventor: Zanelli, Raffaele, 47020 Calabrina di Cesena (FC) (IT)
(74) Representative: Lanzoni, Luciano

(56) References cited:
- EP-A- 0 568 763
- US-A- 3 777 877
- US-A- 4 324 335
- US-A- 4 825 068
- US-B1- 6 267 222
- PATENT ABSTRACTS OF JAPAN vol. 1998, no. 02, 30 January 1998 (1998-01-30) & JP 09 271723 A (TECHNO ISHII:KK), 21 October 1997 (1997-10-21)
- PATENT ABSTRACTS OF JAPAN vol. 1998, no. 09, 31 July 1998 (1998-07-31) & JP 10 111116 A (DAIDO DENKI KOGYO KK), 28 April 1998 (1998-04-28)
- PATENT ABSTRACTS OF JAPAN vol. 1997, no. 12, 25 December 1997 (1997-12-25) & JP 09 215963 A (ISHII IND CO LTD), 19 August 1997 (1997-08-19)
- PATENT ABSTRACTS OF JAPAN vol. 1996, no. 05, 31 May 1996 (1996-05-31) & JP 08 005563 A (SHIRAYANAGISHIKI SENKAKI KK;OTHERS: 02), 12 January 1996 (1996-01-12)

## Description

The present invention relates to an apparatus for optically checking the exterior quality of bulbous fruit and vegetable products of various shapes and sizes. This apparatus can be inserted in a complex machine or in a line for industrial processing and/or for calibrating the weight and dimensions of fruit and vegetable products. The present invention is particularly suitable for checking fruits or the like.

Optical checks of the exterior quality of fruits are used very frequently to classify the fruits themselves in merit categories based on the presence or absence of superficial defects (for instance: rust, nicks, dotting, pests and rotten areas), or to assess their colour and geometric parameters (such as diameter or, in general, the exterior dimensions). Once they are classified, the fruits are then physically subdivided and sorted in groups of homogeneous quality, to be marketed with a price that depends on their quality.

Normally, fruits are conveyed along a path whereon one or more cameras are positioned (black and white or colour, or of both kinds, depending on the type of check to be carried out). These cameras define a field of view within which the fruit passes and which can be observed through the acquisition of one or more images (also at successive times, if necessary) by the cameras. The images are then combined and processed by one or more electronic computers, which identify the superficial defects and classify the fruits.

To enable the analysis of the entire surface of each fruit, known apparatuses for checking exterior quality are provided with means for conveying the fruits which keep the fruits constantly rotating throughout the time necessary for travelling through the area observed by the cameras. This rotation of the fruit takes place about an axis that is horizontal and perpendicular to the direction of the path.

In particular, a chain conveys from the entry to the exit of the apparatus a succession of carriages, each superiorly provided with a pair of parallel rollers. Said two rollers are positioned at a certain distance one from the other, with their axis positioned horizontally and perpendicularly to the direction of motion of the chain. Each carriage can thus convey a fruit while bearing on the two rollers (being partially inserted in the space present between them). The rollers are free to rotate about their axis and are maintained in constant rotation (which is then transmitted to the fruit) by a motorised belt, which intercepts the surface of the rollers throughout the duration of the travel of the carriage underneath the cameras. The belt moves in agreement with the chain. The ratio between the velocity of motion of the belt and the velocity of motion of the chain determines the velocity and direction of rotation of the rollers and, thus, of the fruit set down thereon.

During the continuous rotation of the fruit, the cameras thus acquire, at successive times, images which represent different (but partially superposed) portions of the surface of the fruit, whose union represents the entire surface of the fruit. The electronic computer then clears doubled parts (due to superposition) from said union and identifies and classifies defects.

These apparatuses for optically checking the exterior quality of fruit and vegetable products (in particular fruits) have some drawbacks.

In particular, they exhibit limitations when they have to operate at high checking rates, i.e. to check a high number of fruits per unit of time. A high checking rate, infact, equates to a high velocity of the conveying means, so that each fruit travels over a considerable distance in the unit of time and tends rapidly to depart the field of view of the cameras. In order to be viewed completely, each fruit must undergo a complete rotation during the time interval in which it remains within the field of view of the cameras. When the checking rate is high, the rotation of the fruit must be very rapid and this greatly reduces the precision of the observations and the definition of the images.

Moreover if, as often occurs, the fruit or vegetable product is not very regular, when it is subjected to such a rapid rotation on the two support rollers it may start to bounce or even be ejected from the carriage. This creates obvious problems with the precision of the optical check and/or the repeatability of the positioning of the fruit under the cameras. Some particularly flattened fruits even end up not rotating at all or rotating in fits and starts.

To try to maintain a good quality of the images and, at the same time, to avoid the aforementioned mechanical problems, one is forced to work at low checking rates or to increase, in proportion to the checking frequency, both the number of cameras used and the length of the path underneath the cameras themselves, with obvious problems, respectively, with the management of the data processing operation and of the dimensions of the apparatus.

Optical checking apparatuses are normally integrated in lines for processing fruit and vegetable products or fruits. Such processing lines can have a high processing rate in terms of products processed per second, especially in the case of not particularly delicate products. The drawbacks of known optical checking apparatuses therefore can either create a bottleneck for the productivity of the line or, if the efficiency of the checking apparatus is to match the productivity of the line, they can cause an increase in the costs or the dimensions of the plant.

The abstract of document JP09271723 discloses a series of rotors which constitute a classifying conveyor of fruit vegetables especially oranges. These rotors are rotated and returned to a horizontal position and circulated and transferred in the feeding direction to convey oranges. By some image pickup cameras, a portion of the surface is imaged and stored in a judgment device; a not better defined fruit vegetables reversing machine turns oranges and the complementary portion of the surface of oranges is imaged. Based on the picture data imaged, the rotors are oscillated to discharge and classify oranges by grade.

The abstract of document JP09215963 discloses a series of crossing frames which constitute a classifying conveyor. A large number of oranges is placed on the respective crossing frames. Four placing rollers supported on each of the crossing frames are rotated and in this way each mandarine orange placed on each of the crossing frames is rotated in order to photograph all the surfaces of the mandarine oranges. The grade of each of the mandarine oranges is judged on the basis of the image data taken by a photographing camera by a grade judging device. Each of the mandarine oranges is moved to each distributing position corresponding to judgement.

The abstract of document JP10111116 discloses an inspection device of uneven defects on all-round surface of vegetables and fruits. This device comprises V-shaped belt conveyors which are arranged with their ends arranged in a staggered configuration with spacing. Some rotating rollers are arranged between the ends of the belt conveyors. TV cameras are provided for the space of the V-shape belt conveyors to photograph all round surface of the material. Document US 6 267 222 discloses a device which comprises two viewing posts arranged successively on the transport path of the articles, each post being designed for the inspection of the exposed face resting on the transport means. Turning means for the articles are arranged between the two viewing stations and they pick articles from a first conveyor and transport them on a second conveyor. In fact the turning means comprise two supporting rocker arms, defining between them a holding space for an article in which the article is retained substantially motionless relative to the support arms.

An aim of the present invention is to overcome the aforesaid drawbacks, making available an apparatus for optically checking the exterior quality of bulbous fruit and vegetable products of various shapes and sizes whose performance, in terms of image quality and data processing requirements, depend only to a negligible extent on checking rate. Another aim of the present invention is to provide an apparatus for optically checking the exterior quality of bulbous fruit and vegetable products of various shapes and sizes which is reliable from the mechanical point of view and does not cause problems linked to the positioning of the fruit or vegetable product. These aims and others besides, which shall become more readily apparent from the description that follows, are achieved, in accordance with the present invention, by an apparatus for optically checking the exterior quality of bulbous fruit and vegetable products of various shapes and sizes having structural and functional characteristics in accordance with the accompanying independent claims, additional embodiments thereof being identified in the enclosed and corresponding dependent claims.

The invention is described in closer detail below with the aid of the drawings, which represent embodiments provided purely by way of non limiting examples.
- Figure 1 shows a front schematic view of the apparatus according to an embodiment of the invention, in which the direction of motion of the fruit and vegetable products is indicated by an arrow and in which the parts which cause the upsetting of the products are only indicated schematically with a rectangle drawn in dashed lines.
- Figure 2 shows a plan schematic view of the apparatus of Figure 1, illustrating some details of the area in which the fruit or vegetable product is upset, other details being removed for the sake of further clarity.
- Figure 3 illustrates a side view, partially sectioned in the plane A-A of Figure 2, of the passage of a fruit or vegetable product in a first observation station.
- Figure 4 illustrates a side view, partially sectioned in the plane B-B of Figure 2, of the passage of a fruit or vegetable product in the area in which the fruit or vegetable is upset.
- Figure 5 illustrates a side view, partially sectioned in the plane C-C of Figure 2, of the completed upsetting of the fruit or vegetable product.
- Figure 6 illustrates a side view, partially sectioned in the plane D-D of Figure 2, of the passage of a fruit or vegetable product in a second observation station.
- Figure 7 illustrates a detail of a second support of the fruit and vegetable products, containing a product and partially sectioned and viewed according to the direction designated as E in Figure 5, the direction of motion of the support along the path of the fruit and vegetable products being indicated by the arrow.
- Figure 8 illustrates a variation of the first observation station, seen from a direction of view similar to that of Figure 3.
- Figure 9 illustrates a plan view of a variation of the second observation station.
- Figure 10 illustrates a plan view of an additional variation of the first observation station.

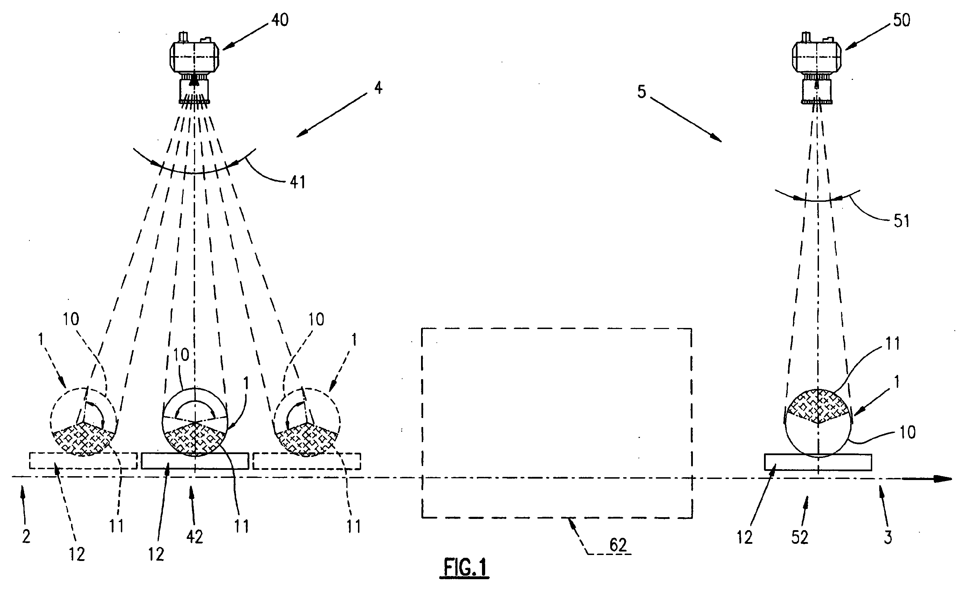

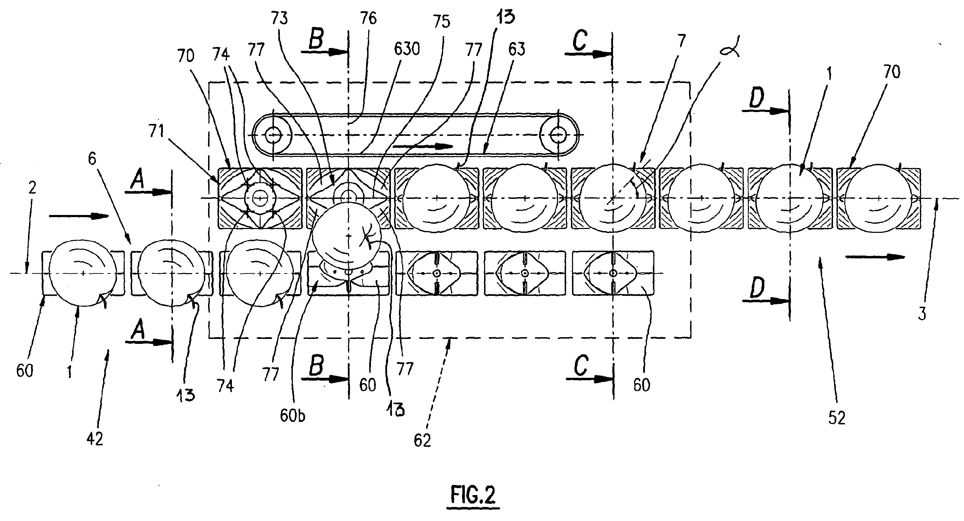

With reference to the figures, an apparatus for optically checking the exterior quality of bulbous fruit and vegetable products of various shapes and sizes comprises means 12 for conveying a succession of fruit and vegetable products 1 along a path from an entry 2 to an exit 3 of the apparatus. A possible path is schematically shown with dashed lines and with an arrow in Figure 1, whilst in Figure 2 some arrows indicate the direction of travel followed by the fruit and vegetable products 1.

A first observation station 4 is positioned along the path and is provided with at least a primary camera 40 in such a way as to define a primary field of view 41 for the observation of a first segment 42 of the path. A second observation station 5 is positioned along the path downstream of the first observation station 4 and is provided with at least a secondary camera 50 in such a way as to define a secondary field of view 51 for the observation of a second segment 52 of the path. The second segment 52 is separate from the first segment 42. The apparatus further comprises means for upsetting the fruit and vegetable products 1 structured in such a way as to act exclusively in a third segment 62 of the path interposed between the first and the second segment 42, 52, therein upsetting each fruit or vegetable product 1 from a first position, in which a first surface portion 10 of the fruit or vegetable product 1 falls within the primary field of view 41 when the fruit or vegetable product 1 is in the first segment 42 of the path, to a second position, in which a second surface portion 11 of the fruit or vegetable product 1, at least complementary to the first surface portion 10, falls within the secondary field of view 51 when the fruit or vegetable product is in the second segment 52 of the path. In Figures 1, 3-6, 8 the second surface portion 11 is highlighted with double crossed dashes, the complementary, not dashed, part representing the first surface portion 10. In Figure 2, the upsetting operation whereto the fruit or vegetable product 1 is subjected in the third segment 62 by the upsetting means is also indicated by the different orientation assumed by the leaf-stalk 13 of the fruit which therein represents the fruit or vegetable product 1 itself. Each fruit or vegetable product 1 is kept stationary relative to the conveying means 12 along the whole first segment 42 and along the whole second segment 52 of the path and therefore travels through both the primary field of view 41 and the secondary field of view 51 always remaining stationary relative to the conveying means 12. Its upsetting from the first to the second position occurs only in the third segment 62 of the path, outside the range of the primary camera 40 and secondary camera 50. Therefore, the performance of said cameras (and of the system) in terms of image qualify is not influenced, except to a negligible extent, by the checking rate. Since the first and second surface portion 10, 11 of the surface of the fruit or vegetable product 1 are mutually complementary (and can also partially superpose on each other), the two observations, performed respectively in the first and in the second observation station 4, 5, are sufficient to analyse the whole fruit or vegetable product 1.

Within the scope of independent claim 1 the upsetting means could be obtained in different ways, provided they are suitable for the purpose of determining the upsetting of the fruit or vegetable product 1 from the first to the second position only in the third segment 62 of the path and in such a way as to guarantee the positioning of the second surface portion 11 of the fruit or vegetable product 1 always within the secondary field of view 51.

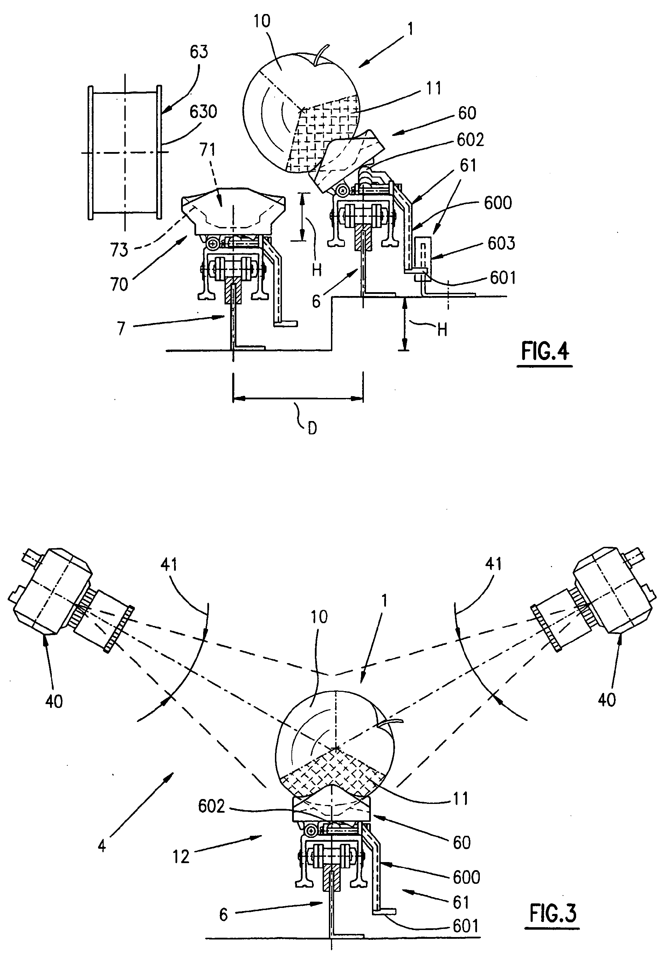

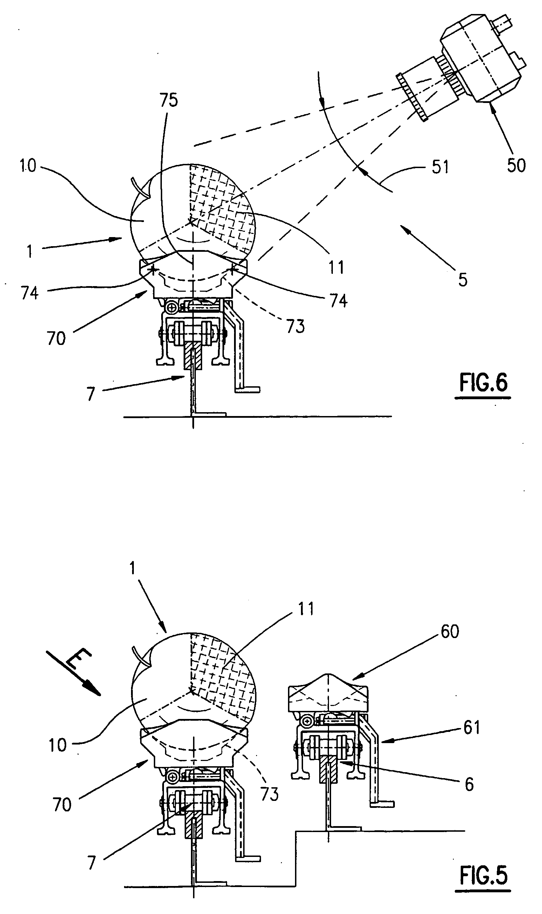

A preferred embodiment of the invention (shown in particular in Figures 2 through 6) allows to obtain a minimisation of the dimensions of the third segment 62, thereby compacting the apparatus, even in case of high checking rates and, at the same time, it allows to avoid the mechanical problems described above. In particular, with reference to Figure 2 (which does not show the primary camera 40 and secondary camera 50), the conveying means 12 of the present invention comprise a first conveying chain 6 which conveys at least along the first and the third segment 42, 62 of the path a succession of first supports 60, each able to support a fruit or vegetable product 1 in the first position and able to be upset by a predetermined angle about an axis parallel to the direction of the path. The conveying means 12 also comprise a second conveying chain 7 that conveys at least along the second and the third segment 52, 62 of the path a succession of second supports 70, each able to support a fruit or vegetable product 1 in the second position. The means for upsetting the fruit or vegetable products 1 comprise actuator means 61 (not shown in Figure 2, but visible in the subsequent Figures 3 through 6) for upsetting the first supports 60 acting in the third segment 62 of the path. Along the third segment 62 of the path, the first and the second conveying chain 6, 7 travel side by side and parallel, maintaining the first supports 60 in correspondence with the second supports 70, the actuators 61 upsetting each first support 60 towards the corresponding second support 70 and determining the transfer thereon of the fruit or vegetable product 1 simultaneously with its upsetting from the first to the second position. In Figure 2 the action of the actuators 61 (not shown) in only the third segment 62 of the path is shown exercised on the first spupport designated with the reference number 60b, the upsetting of the fruit or vegetable product 1 being made visible by the change in position of the leaf stalk 13 of the fruit which in the figure represents the fruit or vegetable product 1 itself. Figures 3 through 6, which represent in succession, respectively, the passage of the fruit or vegetable product 1 in the first observation station 4, its upsetting from the first to the second position in the third segment 62 of the path (Figures 4 and 5) and its passage in the second observation station 5, clearly indicate both the first and the second surface portion 10, 11.

The actuators 61 for upsetting the first supports 60 can be obtained in different manners. A particularly simple and advantageous preferred embodiment thereof, shown in Figures 3 through 6 comprises, for each first support 60, a thrusting element 600 with a first end 601 free and a second end 602 in contact with the first support 60 at a predetermined distance from the axis of upsetting of the first support 60 itself. The actuators 61 for upsetting the first supports 60 further comprise a cam 603 positioned on the third segment 62 of the path in such a way as to abut in succession against the first free end 601 of the thrusting element 600 of each first support 60 and to determine a motion of the thrusting element 600 from a resting position of the first support 60 to an upsetting position of the first support 60, during which the second end 602 thrusts the first support 60 until the upsetting, as well as a return motion to the resting position, with consequent repositioning of the first support 60. The resting position is shown in Figure 3. The motion from the resting position to the upsetting position is shown in Figure 4. Figure 5 illustrates the completed return to the resting position once the first support 60 involved has overtaken the cam 603.

The cam 603 can be constructed in the form of sliding guide having a first wedge-like part, the first free end 601 being a sliding block. The thrusting element 600 can be a lever pivotally engaged on the first conveying chain 6. A detailed description of a support capable of being upset, provided with a form of upsetting actuators of the type described herein can be found in European patent EP 568 763 B1 (although in that context the support for the fruit or vegetable product is used for another purpose).

Advantageously, the profile of the cam 603 has in its initial part, corresponding to the passage from the resting position to the upsetting position, an average slope that is smaller than a predetermined value to allow a smooth upsetting of the fruit or vegetable products 1, minimising the possibility of uncontrolled impulsive movements during the upsetting operation. This guarantees a greater repeatability in the orientation of the second surface portion 11 of the fruit or vegetable product 1 within the secondary field of view 51. When the cam 603 is obtained with a wedge-shaped guide, this effect is obtained by means of a low slope of a first ramp of the wedge-shaped guide.

Appropriately, as shown in Figures 2 and 4, the apparatus further preferably comprises a vertical support surface 63 positioned along the third segment 62 of the path at least in correspondence with the working area of the actuators 61 for upsetting the first supports 60 and set side by side to the second chain 7 on the opposite side relative to the first chain 6. The vertical support surface 63 is obtained with a motorised belt 630, whose movement in the third segment 62 of the path is in agreement with the motion of the first and of the second chain 6, 7 and has the same speed thereof. It thereby becomes impossible for the fruit or vegetable product 1 as a result of the impulse received in the upsetting operation, to depart its second support 70, because it is held laterally to the second transport chain 7 by the vertical support surface 63. It is important for the first and the second conveying chain 6, 7, as well as the belt 630, to travel at the same speed: the fruit or vegetable product 1 thus moves between parts that are stationary relative to each other.

According to the present invention, each second support 70 comprises a cup 71 for housing the fruit or vegetable product 1 whose cavity has inner surfaces 73 so shaped as to provide the fruit or vegetable product 1 with at least four support points 74 arranged by twos at opposite sides relative to two mutually perpendicular vertical planes 75, 76 which pass through the centre of the cup 71.

In this way the fruit or vegetable product 1, falling into the second support 70, always finds secure support points which prevent excessively unpredictable oscillations in the direction of the path and/or in the direction transverse thereto, which could alter the positioning repeatability of the second surface portion 11. In a particular embodiment of the invention, the inner surfaces 73 comprise four pitches 77 with slopes each concurring towards the centre of the cup 71 with a predetermined angle α relative to an axis of the second support 70 directed along the path. A useful value of said angle α is about 45°, but other values can be used depending on working conditions, on the characteristics of the fruit or vegetable product 1 and on requirements.

Advantageously, as shown in particular in Figure 4, along the third segment 62 of the path at least in correspondence with the action of the actuators 61 for the upsetting of the first supports 60, the height H where the first supports 60 are located relative to the second supports 70 and the distance D between the first and the second chain 6, 7 are predetermined in such a way that the upsetting of a fruit or vegetable product 1, whose exterior dimensions (for instance the diameter or diameters) belong to a predetermined interval, always causes the second surface portion 11 to fall within the secondary field of view 51. It is thereby possible to operate without any problems on fruit or vegetable products 1 of various sizes without having first to discriminate, sending different sized fruits to different checking apparatuses.

Both the primary camera 40 and the secondary camera 50 can, respectively within the primary field of view 41 and secondary field of view 51, acquire the images of the related surface portion of fruit or vegetable product 1 (respectively the first 10 and the second 11) with a succession of shots (as shown for instance in the left part of Figure 1 with reference to the first observation station 4, where three different positions are illustrated of a fruit or vegetable product 1 within the primary field of view 41, together with the respective fractions of first surface portion 10 covered by each shot), or with a single shot.

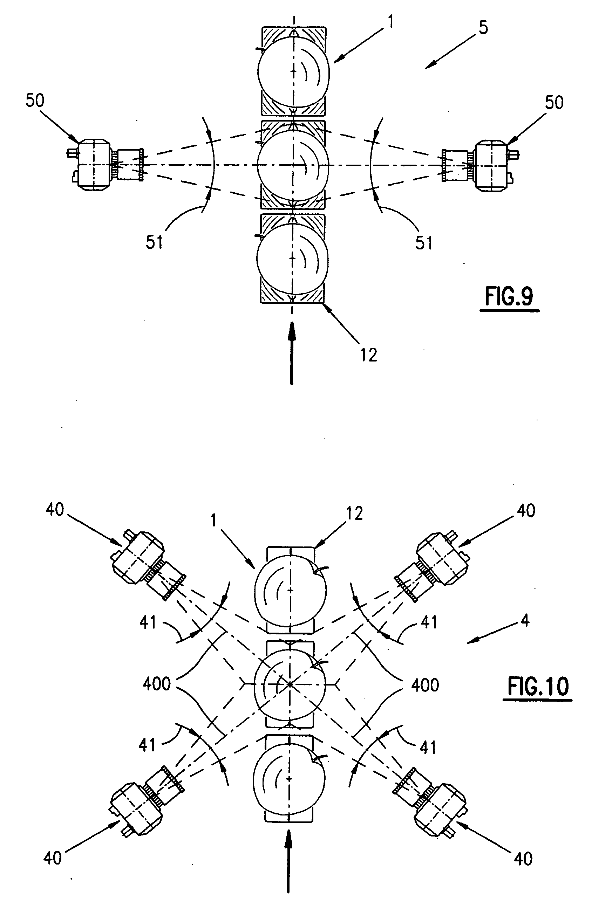

Preferably, to speed up checking and make the architecture more compact in terms of path length, multiple primary cameras 40 and/or multiple secondary cameras 50 can be used. As shown in Figure 3, the first observation station 4 can be provided with at least two primary cameras 40, positioned at opposite sides of a vertical plane containing the first segment 42 of the path, in such a way as to define the primary field of view 41. Similarly, as shown in Figure 9, additionally or alternatively the second observation station 5 can also be provided with at least two secondary cameras 50, positioned at opposite sides of a vertical plane containing the second segment 52 of the path, in such a way as to define the secondary field of view 51. In general, the first observation station 4 can be provided with a plurality of primary cameras 40 distributed in such a way as to define the primary field of view 41. Similarly, additionally or alternatively, the second observation station 5 can be provided with a plurality of secondary cameras 50 distributed in such a manner as to define the secondary field of view 51. A particular configuration of cameras is shown in Figure 10 with reference to the first observation station 4. The first observation station 4 in this case is provided with four primary cameras 40, located two by two at opposite sides of a vertical plane containing the first segment 42 of the path and with optical axes 400 converging on appoint of the path, in such a way as to define the primary field of view 41. Obviously, although it is not shown herein, the same configuration of secondary cameras 50 could be used, additionally or alternatively, also in the second observation station 5.

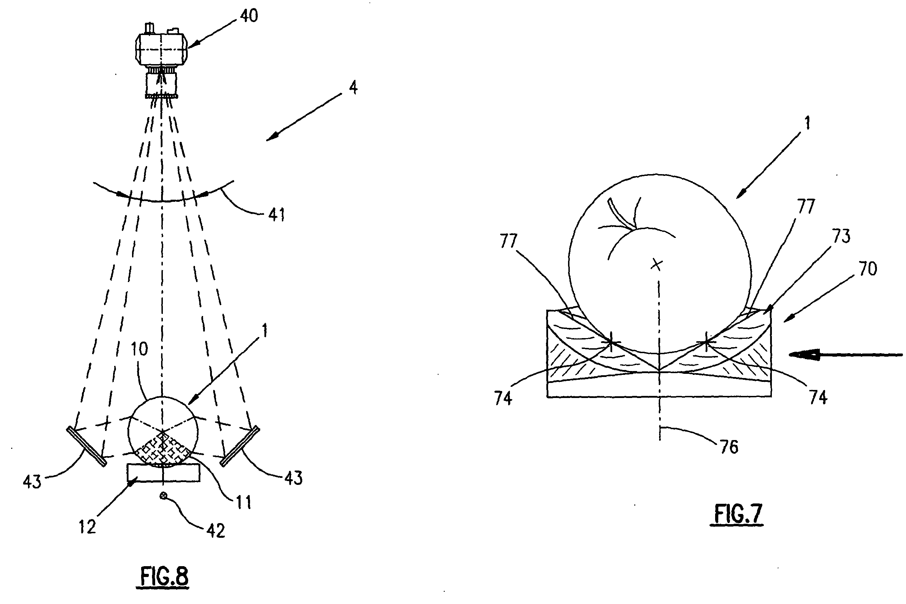

To allow observing the first surface portion 10 of the fruit or vegetable product 1 with the lowest possible number of shots even by a single primary camera 40, the apparatus may comprise at least a mirror 43 operatively associated to the first segment 42 of the path in correspondence with the conveying means 12 on a side of the fruit or vegetable products 1, in such a way as to expand the surface of a fruit or vegetable product 1 that can be observed in the primary field of view 41. Advantageously, as shown in Figure 8, two mirrors 43 can be provided, associated to the first segment 42 of the path on the two sides thereof. Obviously, if necessary, this solution can also be adopted to aid the work of the second observation station 5. The mirrors 43 can be fixed relative to the path, but they could also be conveyed along the path together with the fruit or vegetable products 1.

In general, the apparatus according to the invention can be incorporated in a line for processing bulbous fruit or vegetable products 1 of various shapes and sizes. In particular it can be positioned downstream of a singling machine, i.e. a machine that provides, on the conveying means 12, a succession of single and well identified fruit or vegetable products 1. In this case, for instance, the first conveying chain 6 can coincide with the chain that conveys the fruit or vegetable products 1 out of the singling machine itself. The singling machine may itself be a gauging machine (i.e. a machine capable of selecting the fruit or vegetable products 1 according to weight and/or dimensions). The gauging machine may, alternatively, be positioned downstream of the checking apparatus. In this case, for instance, the second conveying chain 7 can coincide with the chain that conveys the fruit or vegetable products 1 into the gauging machine. The second supports 70 may, moreover, also be capable of being upset for unloading the fruit or vegetable products 1.

In general, with reference to the preferred embodiment of the invention and, in particular, to Figures 2 through 6, during use the fruit or vegetable products 1, each positioned on the corresponding first support 60, are conveyed by the first conveying chain 6, stationary in their first position, within the first observation station 4. Therein, the first surface portion 10 enters the primary field of view and, along the first segment 42 of the path, is analysed by the primary camera 40 (or by the primary cameras 40). Along the third segment 62 of the path, the first conveying chain 6 is side by side with the second conveying chain 7 and the first support 60 is perfectly side by side with a corresponding second support 70 and relatively stationary with respect thereto. The first free end 601 of the thrust element 600 of the first support 60 is abutted by the cam 603 and the first support 60 is upset towards the second support 70 transferring the upset fruit or vegetable product 1 in the second position. The fruit or vegetable product thus positioned leaves, in the second conveying chain 7, the third segment 62 of the path, remaining stationary in its second position, and enters the secondary field of view 51 defined by the distribution of secondary cameras 50 in the second observation station 5. The secondary cameras 50 complete the analysis of the fruit or vegetable product 1 acquiring images of the second surface portion 11. The fruit or vegetable product 1 leaves the apparatus and may be unloaded downstream therefrom.

The invention achieves important advantages. First of all, it enables a high checking rate without problems with image quality and, hence with check reliability. Mechanical problems are eliminated and an extremely compact apparatus is obtained, in which the optical components and computing devices can be minimised. The apparatus can easily be included even in very fast processing lines for fruit or vegetable products, in particular fruits.

All materials employed, as well as dimensions, may be any, depending on requirements.

## Claims

1. An apparatus for optically checking the exterior quality of bulbous fruit and vegetable products of various shapes and sizes, comprising:
- means (12) for conveying a succession of fruit or vegetable products (1) along a path from an entry (2) to an exit (3) of the apparatus;
- a first observation station (4) positioned along the path and provided with at least a primary camera (40) in such a way as to define a primary field of view (41) for the observation of a first segment (42) of the path;
- a second observation station (5) positioned along the path downstream of the first observation station (4) and provided with at least a secondary camera (50) in such a way as to define a secondary field of view (51) for the observation of a second segment (52) of the path, separate from the first segment (42);
- means for upsetting the fruit or vegetable products (1) structured in such a way as to act exclusively in a third segment (62) of the path interposed between the first and the second segment (42, 52), therein upsetting each fruit or vegetable product (1) from a first position, in which a first surface portion (10) of the fruit or vegetable product (1) falls within the primary field of view (41) when the fruit or vegetable product (1) is in the first segment (42) of the path, to a second position, in which a second surface portion (11) of the fruit or vegetable product (1), at least complementary to the first surface portion (10), falls within the secondary field of view (51) when the fruit or vegetable product is in the second segment (52) of the path;
and in which the conveying means (12) comprise:
- a first conveying chain (6) which conveys at least along the first and the third segment (42, 62) of the path a succession of first supports (60), each able to sustain a fruit and vegetable product (1) in the first position and able to be upset by a predetermined angle about an axis parallel to the direction of the path;
- a second conveying chain (7) which conveys at least along the second and the third segment (52, 62) of the path a succession of second supports (70), each able to sustain a fruit or vegetable product (1) in the second position;
and in which:
- the means for upsetting the fruit or vegetable products (1) comprise actuators (61) to upset the first supports (60) acting in the third segment (62) of the path;
- along the third segment (62) of the path the first and the second conveying chain (6, 7) travel side by side and parallel, maintaining the first supports (60) in correspondence with the second supports (70), the actuators (61) upsetting each first support (60) towards the corresponding second support (70) and determining the transfer thereon of the fruit or vegetable product (1) simultaneously with its upsetting from the first to the second position;
said apparatus for optically checking the exterior quality of bulbous fruit and vegetables products being **characterised in that**:
each second support (70) comprises a cup (71) for housing the fruit or vegetable product (1) whose cavity has inner surfaces (73) so shaped as to provide the fruit or vegetable product (1) with at least four support points (74) arranged by twos at opposite sides relative to two mutually perpendicular vertical planes (75, 76) which pass through the centre of the cup (71), the fruit or vegetable product (1) falling into the second support (70).

2. An apparatus as claimed in claim 1, **characterised in that**, along the third segment (62) of the path at least in correspondence with the action of the actuators (61) upsetting the first supports (60), the height (H) whereat the first supports (60) are located relative to the second supports (70) and the distance (D) between the first and the second chain (6, 7) are predetermined in such a way that the upsetting of a fruit or vegetable product (1) whose exterior dimensions belong to a predetermined range always causes the second surface portion (11) to fall within the secondary field of view (51).

3. An apparatus as claimed in claim 1 or 2, **characterised in that** it comprises a vertical support surface (63) positioned along the third segment (62) of the path at least in correspondence with the area of action of the actuators (61) upsetting the first supports (60) and set side by side to the second chain (7) on the opposite side from the first chain (6), the vertical support surface (63) being obtained with a motorised belt (630), whose motion in the third segment (62) of the path is in agreement with the motion of the first and of the second chain (6, 7) and has the same velocity thereof.

4. An apparatus as claimed in any of the previous claims **characterised in that** the inner surfaces (73) comprise four pitches (77) with slopes each concurring towards the centre of the cup (71) with a predetermined angle (α) relative to an axis of the second support (70) directed along the path.

5. An apparatus as claimed in any of the previous claims, **characterised in that** the actuators (61) for upsetting the first supports (60) comprise:
- for each first support (60), a thrusting element (600) with a first end (601) free and a second end (602) in contact with the first support (60) at a predetermined distance from the upset axis of the first support (60) itself;
- a cam (603) positioned on the third segment (62) of the path in such a way as to abut in succession the first free end (601) of the thrusting element (600) of each first support (60) and determine a motion of the thrusting element (600) from a resting position of the first support (60) to an upset position of the first support (60), during which the second end (602) thrusts the first support (60) until its upsetting, with consequent repositioning of the first support (60).

6. An apparatus as claimed in claim 5 **characterised in that** the profile of the cam (603) has in its initial part, corresponding to the passage from the resting position to the upset position, an average slope that is smaller than a predetermined value to allow a smooth upsetting of the fruit or vegetable products (1).

7. An apparatus as claimed in any of the previous claims, **characterised in that** it comprises at least a mirror (43) operatively associated to the first segment (42) of the path in correspondence with the conveying means (12) on a side of the fruit or vegetable products (1) in such a way as to expand the surface of a fruit or vegetable product (1) that can be observed in the primary field of view (41).

8. An apparatus as claimed in claim 7, **characterised in that** it comprises two mirrors (43) associated to the first segment (42) of the path on the two sides thereof.

9. An apparatus as claimed in any of the previous claims, **characterised in that** the first observation station (4) is provided with at least two primary cameras (40), positioned at opposite sides of a vertical plane containing the first segment (42) of the path, in such a way as to define the primary field of view (41).

10. An apparatus as claimed in any of the claims from 1 to 8, **characterised in that** the first observation station (4) is provided with a plurality of primary cameras (40) in such a way as to define the primary field of view (41).

11. An apparatus as claimed in claim 10, **characterised in that** the first observation station (4) is provided with four primary cameras (40), located two by two at opposite sides of a vertical plane containing the first segment (42) of the path and with optical axes (400) converging on a point of the path, in such a way as to define the primary field of view (41).

12. An apparatus as claimed in any of the previous claims, **characterised in that** the second observation station (5) is provided with at least two secondary cameras (50), positioned at opposite sides of a vertical plane containing the second segment (52) of the path, in such a way as to define the secondary field of view (51).

13. An apparatus as claimed in any of the claims from 1 to 11, **characterised in that** the second observation station (5) is provided with a plurality of secondary cameras (50) distributed in such a way as to define the secondary field of view (51).

14. Line for processing bulbous fruit or vegetable products (1) of various shapes and sizes, **characterised in that** it comprises an apparatus for optically checking the exterior quality of fruit or vegetable products (1) as claimed in any of the previous claims.

## Patentansprüche

1. Gerät zur optischen Überprüfung der äusseren Qualität von gewölbt geformten Obst- und Gemüseprodukten von verschiedenen Formen und Grössen, enthaltend:
- Mittel (12) zum Transportieren einer Folge von Obst- oder Gemüseprodukten (1) entlang einer Bahn von einem Eingang (2) zu einem Ausgang (3) des Gerätes;
- eine erste Beobachtungsstation (4), positioniert entlang der Bahn und versehen mit wenigstens einer primären Kamera (40) auf solche Weise, dass ein primäres Sichtfeld (41) zur Beobachtung eines ersten Abschnittes (42) der Bahn festgelegt wird;
- eine zweite Beobachtungsstation (5), positioniert entlang der Bahn stromabwärts der ersten Beobachtungsstation (4), versehen mit wenigstens einer sekundären Kamera (50) auf solche Weise, dass ein sekundäres Sichtfeld (51) zur Beobachtung eines zweiten Abschnittes (52) der Bahn festgelegt wird, getrennt von dem ersten Abschnitt (42);
- Mittel zum Umdrehen der Obst- oder Gemüseprodukte (1), die auf solche Weise strukturiert sind, dass sie ausschliesslich in einem dritten Abschnitt (62) der Bahn wirken, der zwischen dem ersten und dem zweiten Abschnitt (42, 52) eingesetzt ist und hierin jedes Obst- oder Gemüseprodukt (1) umdreht aus einer ersten Position, in welcher ein erster Oberflächenabschnitt (10) des Obst- oder Gemüseproduktes (1) innerhalb des ersten Sichtfeldes (41) liegt, wenn das Obst- oder Gemüseprodukt (1) sich in dem ersten Abschnitt (42) der Bahn befindet, in eine zweite Position, in welcher ein zweiter Oberflächenabschnitt (11) des Obst- oder Gemüseproduktes (1), der wenigstens ergänzend zu dem ersten Oberflächenabschnitt (10) ist, innerhalb des zweiten Sichtfeldes (51) liegt, wenn das Obst- oder Gemüseprodukt (1) sich in dem zweiten Abschnitt (52) der Bahn befindet;
und bei welchem die Transportmittel (12) enthalten:
- eine erste Transportkette (6), welche wenigstensentlang dem ersten und dem dritten Abschnitt (42, 62) der Bahn eine Folge von ersten Halterungen (60) transportiert, jede in der Lage, ein Obst- und Gemüseprodukt (1) in der ersten Position zu halten und geeignet, um einen vorgegebenen Winkel um eine Achse parallel zu der Richtung der Bahn gekippt zu werden;
- eine zweite Transportkette (7), welche wenigstens entlang dem zweiten und dritten Abschnitt (52, 62) der Bahn eine Folge von zweiten Halterungen (70) transportiert, jede in der Lage, ein Obst- oder Gemüseprodukt (1) in der zweiten Position zu halten;
und bei welchem:
- die Mittel zum Umdrehen der Obst- oder Gemüseprodukte (1) Stellantriebe (61) zum Kippen der ersten Halterungen (60) enthalten, die in dem dritten Abschnitt (62) der Bahn wirken;
- entlang dem dritten Abschnitt (62) der Bahn die erste und die zweite Transportkette (6, 7) Seite an Seite und parallel zueinander laufen, wobei die ersten Halterungen (60) entsprechend zu den zweiten Halterungen (70) gehalten sind, und wobei die Stellantriebe (61) jede erste Halterung (60) zu der entsprechenden zweiten Halterung (70) hin kippen können und somit das Übertragen auf diese des Obst- oder Gemüseproduktes (1) gleichzeitig mit dessen Umdrehen aus der ersten in die zweite Position bewirken;
wobei das genannte Gerät zur optischen Überprüfung der äusseren Qualität von gewölbt geformten Obst- und Gemüseprodukten **dadurch gekennzeichnet ist, dass**:
jede zweite Halterung (70) eine Schale (71) zur Aufnahme des Obst- oder Gemüseproduktes (1) enthält, deren innere Oberflächen (73) so ausgeformt sind, dass für das Obst- oder Gemüseprodukt (1) mindesten vier Haltepunkte (74) vorgesehen sind, angeordnet zu zweien auf gegenüberliegenden Seiten im Verhältnis zu zwei zueinander lotrechten vertikalen Ebenen (75, 76), welche durch die Mitte der Schale (71) verlaufen, während das Obst- oder Gemüseprodukt (1) in die zweite Halterung (70) fällt.

2. Gerät nach Patentanspruch 1, **dadurch gekennzeichnet, dass** entlang dem dritten Abschnitt (62) der Bahn, wenigstens entsprechend dem Wirkungsbereich der Stellantriebe (61), welche die ersten Halterungen (60) kippen, die Höhe (H), auf welcher die ersten Halterungen (60) im Verhältnis zu den zweiten Halterungen (70) angeordnet sind, und der Abstand (D) zwischen der ersten und der zweiten Kette (6, 7), auf solche Weise festgelegt sind, dass das Umdrehen eines Obst- oder Gemüseproduktes (1), dessen äussere Abmessungen zu einem vorgegebenen Bereich gehören, immer das Fallen des zweiten Oberflächenabschnittes (11) in das sekundäre Sichtfeld (51) bewirkt.

3. Gerät nach Patentanspruch 1 oder 2, **dadurch gekennzeichnet, dass** es eine vertikale Stützfläche (63) enthält, positioniert entlang dem dritten Abschnitt (62) der Bahn und wenigstens entsprechend einem Wirkungsbereich der die ersten Halterungen (60) kippenden Stellantriebe (61), und angeordnet Seite an Seite mit der zweiten Kette (7) auf der gegenüberliegenden Seite von der ersten Kette (6), wobei die vertikale Stützfläche (63) durch ein angetriebenes Band (630) erhalten ist, dessen Bewegung in dem dritten Abschnitt (62) der Bahn mit der Bewegung der ersten und der zweiten Kette (6, 7) übereinstimmt, und welches dieselbe Geschwindigkeit wie diese hat.

4. Gerät nach einem beliebigen der vorstehenden Patentansprüche, **dadurch gekennzeichnet, dass** die inneren Oberflächen (73) vier Schrägen (77) enthält, welche übereinstimmend zur Mitte der Schale (71) hin abfallen, und zwar mit einem bestimmten Winkel (α) im Verhältnis zu einer Achse der zweiten, entlang der Bahn laufenden Halterung (70).

5. Gerät nach einem beliebigen der vorstehenden Patentansprüche, **dadurch gekennzeichnet, dass** die Stellantriebe (61) zum Kippen der ersten Halterungen (60) enthalten:
- für jede erste Halterung (60) ein Schubelement (600) mit einem ersten freien Ende (601) und einem zweiten Ende (602) im Kontakt mit der ersten Halterung (60) in einem bestimmten Abstand von der Kippachse der ersten Halterung (60) selbst;
- eine Nocke (603), angeordnet an dem dritten Abschnitt (62) der Bahn auf solche Weise, dass sie aufeinanderfolgend gegen das erste freie Ende (601) des Schubelementes (600) einer jeder ersten Halterung (60) schlägt und eine Bewegung des Schubelementes (600) aus einer Ruheposition der ersten Halterung (60) in eine Kippposition der ersten Halterung (60) bewirkt, während welcher das zweite Ende (602) die erste Halterung (60) bis zu ihrem Kippen schiebt, und zwar mit der anschliessenden erneuten Positionierung der ersten Halterung (60).

6. Gerät nach Patentanspruch 5, **dadurch gekennzeichnet, dass** das Profil der Nocke (603) an seinem Anfangsteil, welcher dem Übergang von der Ruheposition in die Kippposition entspricht, ein durchschnittliches Gefälle aufweist, das geringer ist als ein vorgegebener Wert, um ein weiches Umdrehen der Obst- oder Gemüseprodukte (1) zu erlauben.

7. Gerät nach einem beliebigen der vorstehenden Patentansprüche, **dadurch gekennzeichnet, dass** es wenigstens einen Spiegel (43) enthält, betrieblich zugeordnet dem ersten Abschnitt (42) der Bahn entsprechend den Mitteln (12) zum Transport d er Obst- o der Gemüseprodukte (1) auf ihrer einen Seite, und zwar auf solche Weise, dass die Oberfläche eines in dem primären Sichtfeld (41) zu beobachtenden Obst-oder Gemüseproduktes (1) vergrössert werden kann.

8. Gerät nach Patentanspruch 7, **dadurch gekennzeichnet, dass** es zwei Spiegel (43) enthält, zugeordnet dem ersten Abschnitt (42) der Bahn an beiden Seiten derselben.

9. Gerät nach einem beliebigen der vorstehenden Patentansprüche, **dadurch gekennzeichnet, dass** die erste Beobachtungsstation (4) mit wenigstens zwei primären Kameras (40) versehen ist, positioniert auf sich gegenüberliegenden Seiten einer vertikalen, den ersten Abschnitt (42) der Bahn enthaltenden Ebene, und zwar auf solche Weise, dass das primäre Sichtfeld (41) beschrieben wird.

10. Gerät nach einem beliebigen der Patentansprüche von 1 bis 8, **dadurch gekennzeichnet, dass** die erste Beobachtungsstation (4) mit einer Anzahl von primären Kameras (40) versehen ist, und zwar auf solche Weise, dass das primäre Sichtfeld (41) beschrieben wird.

11. Gerät nach Patentanspruch 10, **dadurch gekennzeichnet, dass** die erste Beobachtungsstation (4) mit vier primären Kameras (40) versehen ist, angeordnet zu zweien auf sich gegenüberliegenden Seiten einer vertikalen, den ersten Abschnitt (42) der Bahn enthaltenden Ebene, und mit optischen Achsen (400), die zu einem Punkt der Bahn auf solche Weise konvergieren, dass das primäre Sichtfeld (41) beschrieben wird.

12. Gerät nach einem beliebigen der vorstehenden Patentansprüche, **dadurch gekennzeichnet, dass** die zweite Beobachtungsstation (5) mit wenigstens zwei sekundären Kameras (50) versehen ist, positioniert auf sich gegenüberliegenden Seiten einer vertikalen, den zweiten Abschnitt (52) der Bahn enthaltenden Ebene auf solche Weise, dass das sekundäre Sichtfeld (51) beschrieben wird.

13. Gerät nach einem beliebigen der Patentansprüche 1 bis 11, **dadurch gekennzeichnet, dass** die zweite Beobachtungsstation (5) mit einer Anzahl von sekundären Kameras (50) versehen ist, verteilt auf solche Weise, dass das sekundäre Sichtfeld (51) beschrieben wird.

14. Anlage zur Verarbeitung von gewölbten Obst- oder Ge-müseprodukten (1) von verschiedenen Formen und Grössen, **dadurch gekennzeichnet, dass** sie ein Gerät zur optischen Überprüfung der äusseren Qualität von Obst- oder Gemüseprodukten enthält, wie in einem jeden der vorstehenden Patentansprüche beansprucht ist.

## Revendications

1. Dispositif de contrôle optique de la qualité extérieure de fruits et légumes bulbeux de forme et de taille diverses, comprenant:
- des moyens (12) de convoyage d'une succession de fruits et légumes (1) le long d'un parcours d'une entrée (2) à une sortie (3) du dispositif;
- une première station d'observation (4) positionnée le long du parcours et pourvue d'au moins une caméra primaire (40) de manière à définir un champ de vision primaire (41) pour l'observation d'un premier segment (42) du parcours;
- une seconde station d'observation (5) positionnée le long du parcours en aval de la première station d'observation (4) et pourvue d'au moins une caméra secondaire (50) de manière à définir un champ de vision secondaire (51) pour l'observation d'un second segment (52) du parcours, séparé du premier segment (42);
- des moyens de renversement des fruits et légumes (1) structurés pour agir exclusivement dans un troisième segment (62) du parcours interposé entre le premier et le second segment (42, 52), dans lequel ils renversent chaque fruit ou légume (1) d'une première position, dans laquelle une première portion de surface (10) du fruit ou légume (1) tombe dans le champ de vision primaire (41) lorsque le fruit ou légume (1) se trouve dans le premier segment (42) du parcours, à une seconde position, dans laquelle une seconde portion de surface (11) du fruit ou légume (1), au moins complémentaire de la première portion de surface (10), tombe dans le champ de vision secondaire (51) lorsque le fruit ou légume se trouve dans le second segment (52) du parcours;
et dans lequel les moyens de convoyage (12) comprennent:
- une première chaîne de convoyage (6) qui convoie au moins le long du premier et du troisième segment (42, 62) du parcours une succession de premiers supports (60), chacun étant capable de soutenir un fruit et légume (1) dans la première position et pouvant être renversé selon un angle prédéterminé autour d'un axe parallèle à la direction du parcours;
- une seconde chaîne de convoyage (7) qui convoie au moins le long du second et du troisième segment (52, 62) du parcours une succession de seconds supports (70), chacun étant capable de soutenir un fruit ou légume (1) dans la seconde position;
et dans lequel:
- les moyens de renversement des fruits ou légumes (1) comprennent des actionneurs (61) pour renverser les premiers supports (60) agissant dans le troisième segment (62) du parcours;
- le long du troisième segment (62) du parcours de la première et seconde chaîne de convoyage (6, 7) voyagent côte à côte et parallèles, en maintenant les premiers supports (60) en correspondance des seconds supports (70), les actionneurs (61) renversant chaque premier support (60) vers le second support correspondant (70) et déterminant le transfert sur ce dernier du fruit ou légume (1) simultanément à son renversement de la première à la seconde position;
ledit dispositif de contrôle optique de la qualité extérieure de fruits et légumes bulbeux étant **caractérisé en ce que**:
chaque second support (70) comprend une coupe (71) pour recevoir le fruit ou légume (1) dont la cavité présente des surfaces internes (73) conformées de manière à fournir au fruit ou légume (1) au moins quatre points de support (74) disposés par paires sur des côtés opposés par rapport à deux plans verticaux (75, 76) mutuellement perpendiculaires qui passent par le centre de la coupe (71), le fruit ou légume (1) tombant dans le second support (70).

2. Dispositif selon la revendication 1, **caractérisé en ce que**, le long du troisième segment (62) du parcours au moins en correspondance de l'action des actionneurs (61) renversant les premiers supports (60), la hauteur (H) à laquelle les premiers supports (60) sont situés par rapport aux seconds supports (70) et la distance (D) entre la première et la seconde chaîne (6, 7) sont prédéterminées de manière à ce que le renversement d'un fruit ou légume (1) dont la dimension externe est comprise dans un intervalle prédéterminé se fasse toujours de manière à ce que la seconde portion de surface (11) se trouve dans le champ de vision secondaire (51).

3. Dispositif selon la revendication 1 ou 2, **caractérisé en ce qu'**il comprend une surface de support verticale (63) positionnée le long du troisième segment (62) du parcours au moins en correspondance de la zone d'action des actionneurs (61) renversant les premiers supports (60) et disposée côte à côte par rapport à la seconde chaîne (7) sur le côté opposé de la première chaîne (6), la surface de support verticale (63) étant obtenue avec une courroie motorisée (630), dont le mouvement dans le troisième segment (62) du parcours concorde avec le mouvement de la première et de la seconde chaîne (6, 7) et présente la même vitesse.

4. Dispositif selon n'importe laquelle des revendications précédentes, **caractérisé en ce que** les surfaces internes (73) comprennent quatre pentes (77) avec des inclinaisons concourrant chacune vers le centre de la coupe (71) selon un angle (α) par rapport à un axe du second support (70) dirigé le long du parcours.

5. Dispositif selon n'importe laquelle des revendications précédentes, **caractérisé en ce que** les actionneurs (61) de renversement des premiers supports (60) comprennent:
- pour chaque premier support (60), un élément de poussée (600) avec une première extrémité (601) libre et une seconde extrémité (602) en contact avec le premier support (60) à une distance prédéterminée de l'axe de renversement du premier support (60) lui-même;
- une came (603) positionnée sur le troisième segment (62) du parcours de manière à buter en succession sur les extrémités libres (601) de l'élément de poussée (600) de chaque premier support (60) et déterminer un mouvement de l'élément de poussée (600) d'une position de repos du premier support (60) à une position de renversement du premier support (60), pendant lequel la seconde extrémité (602) pousse le premier support (60) jusqu'à ce qu'il se renverse, avec un conséquent repositionnement du premier support (60).

6. Dispositif selon la revendication 5, **caractérisé en ce que** le profil de la came (603) présente sur sa partie initiale, correspondant au passage de la position de repos à la position de renversement, une inclinaison moyenne inférieure à une valeur prédéterminée pour permettre un léger renversement du fruit ou légume (1).

7. Dispositif selon n'importe laquelle des revendications précédentes, **caractérisé en ce qu'**il comprend au moins un miroir (43) associé de manière opérationnelle au premier segment (42) du parcours en correspondance des moyens de convoyage (12) sur un côté du fruit ou légume (1) de manière à augmenter la surface d'un fruit ou légume (1) qui peut être observée dans le champ de vision primaire (41).

8. Dispositif selon la revendication 7, **caractérisé en ce qu'**il comprend deux miroirs (43) associés au premier segment (42) du parcours sur ses deux côtés.

9. Dispositif selon n'importe laquelle des revendications précédentes, **caractérisé en ce que** la première station d'observation (4) est pourvue d'au moins deux caméras primaires (40), positionnées sur les côtés opposés d'un plan vertical contenant le premier segment (42) du parcours, de manière à définir le champ de vision primaire (41).

10. Dispositif selon n'importe laquelle des revendications 1 à 8, **caractérisé en ce que** la première station d'observation (4) est pourvue d'une pluralité de caméras primaires (40) de manière à définir le champ de vision primaire (41).

11. Dispositif selon la revendication 10, **caractérisé en ce que** la première station d'observation (4) est pourvue de quatre caméras primaires (40), disposées en paires sur les côtés opposés d'un plan vertical contenant le premier segment (42) du parcours et d'axes optiques (400) convergeant en un point du parcours, de manière à définir le champ de vision primaire (41).

12. Dispositif selon n'importe laquelle des revendications précédentes, **caractérisé en ce que** la seconde station d'observation (5) est pourvue d'au moins deux caméras secondaires (50), positionnées sur des côtés opposés d'un plan vertical contenant le second segment (52) du parcours, de manière à définir le champ de vision secondaire (51).

13. Dispositif selon n'importe laquelle des revendications 1 à 11, **caractérisé en ce que** la seconde station d'observation (5) est pourvue d'une pluralité de caméras secondaires (50) distribuées de manière à définir le champ de vision secondaire (51).

14. Ligne de traitement de fruits ou légumes bulbeux (1) de formes et dimensions variées, **caractérisée en ce qu'**elle comprend un dispositif de contrôle optique de la qualité extérieure de fruits et légumes bulbeux (1) selon n'importe laquelle des revendications précédentes.
